# EUROPEAN PATENT SPECIFICATION

(11) **EP 3 733 145 B1**
(45) Date of publication and mention of the grant of the patent: **04.05.2022**
(21) Application number: 20182110.5
(22) Date of filing: 27.02.2017
(51) Int. Cl.: A61H 1/02, A61F 5/01

(54) **EXOSKELETON DEVICE FOR THE HAND**
EXOSKELETTVORRICHTUNG FÜR DIE HAND
DISPOSITIF D'EXOSQUELETTE POUR LA MAIN

(30) Priority: 26.02.2016 IT UB20161088
(43) Date of publication of application: 04.11.2020
(62) Divisional of application: 17717229.3
(73) Proprietor: Scuola Superiore di Studi Universitari e di Perfezionamento Sant'Anna, 56127 Pisa (IT)
(72) Inventor: SARAC STROPPA, Mine, 56123 Pisa (IT); SOLAZZI, Massimiliano, 56021 Cascina Pisa (IT); SOTGIU, Edoardo, 56038 Ponsacco Pisa (IT); BERGAMASCO, Massimo, 55050 San Michele in Escheto Lucca (IT); FRISOLI, Antonio, 56017 San Giuliano Terme Pisa (IT); GABARDI, Massimiliano, 39012 Merano (IT)
(74) Representative: Celestino, Marco

(56) References cited:
- WO-A1-2015/095459
- ES-A1- 2 558 024
- US-A1- 2010 305 717

## Description

### Field of the invention

The present invention relates to the field of wearable exoskeletons for neuro-motor and orthopedic rehabilitation assisted by robot and for the guidance and assistance to the movement of the limbs during the course of everyday activities.

In particular, the invention relates to an exoskeleton for rehabilitation, the guidance and the assistance to the movement of flexion/extension of the fingers of a hand, also during the cylindrical gripping of objects.

### Description of the prior art

As well known, a problem faced by many mechanical devices wearable (haptic or exoskeleton devices) is to provide an outer kinematic coupling with the human hand and at the same time allowing an exchange of physic actions (forces/torques) with the phalanxes of the hand itself: all this in conditions of efficiency and of maximum safety for the human user. One reason for inefficiency in the transmission of forces from the exoskeletal structure to the phalanx can be represented by the implementation of points of attachment by means of contact surfaces with the phalanx that require the transmission of forces by friction tangentially to the same contact surfaces. The transmission of the motion by means of these frictional forces can be inefficient due to possible sliding between exoskeletal structure and phalanx, both for possible deformations of the skin of the phalanx, both for a coefficient of friction not appropriate for the extent of the forces that are acting. Moreover, this type of transmission of motion can be uncomfortable because of a deformation or a slight abrasion of the skin.

In the more restrictive case of care and rehabilitation, where typically there is a muscular hypertonia during the movements of opening of the hand and consequent resistance offered to the movement, the exoskeleton structure must be able to withstand substantial external loads and transmit them in a comfortable, safe and efficient way to the phalanges and to the articular joints, without causing overloads on the human joints.In fact, the coupling of the human kinematic structure with a sufficiently rigid exoskeletal structure can lead to a non-efficient functioning of the mechanism, as a result of various factors, such as misalignment of the axes of robotic joints with human joints and the variability of the outer conformation of human limb.The particular case of the hand presents these problems in a very extended form, because of its large mobility and small size of its segments. However, the search for solutions to this problem is very desirable in the rehabilitative clinical environments, where the therapies mediated by robotic devices are increasingly being used for their great potential: at the technical level, this need is realized in the request of wearable mechanical devices that allow assistance to individual anatomical joints of the patient's fingers, without interfering with their natural movements and without causing undue discomfort.

The greatest interest is focused on the functional rehabilitation of the grips, such as the cylindrical grip or the precision grip. For this reason, it is necessary that the device should not interfere with the hand palm area, as well as it should allow a control of the patient's wrist flexion to maintain the posture of the hand relative to the arm during the anatomically correct grip. Furthermore, the use of underactuated systems allows to modulate the distribution of the forces on the various phalanges so as to adapt to the grasped object geometry, although using a limited number of actuators to reduce the weight of the device worn.

The document WO2014033613 describes an exoskeleton for rehabilitation of the metacarpal-phalangeal joint of the hand, in particular the thumb and the forefinger. In particular, the exoskeleton above allows the assistance to the flexion-extension of joints of a hand by means of pure torques which do not generate parasitic forces on the phalangeal and metacarpal-phalangeal joints, moving them for their entire natural workspace. In addition, the exoskeleton is adjustable and adaptable to the different anthropometric measurements of the patient's hand.

However, this exoskeleton has two main critical issues.

The first relates to a reduced accuracy in assisting the gripping of an object. In fact, being connected only with the proximal phalanx of the finger, the exoskeleton is very stiff in the distal phalanges and therefore not very effective during the gripping of complex objects and with dimensions not known a priori.

A second aspect relates to the presence of perpendicular reactions on the joints, on the plane of flexion-extension, in correspondence of the connection between the exoskeleton and phalanx. Although reduced, these reactions may cause a limitation to the movement of the fingers and, in the long run, also the onset of articular damages.

The documents ES2558024 and US20100305717 also have two exoskeleton devices for hand rehabilitation.

However, in both documents use is made of circular guides that define the axis of rotation of the phalanges. This implies that, for a proper operation, the center of the guides must coincide with the metacarpo/phalangeal joints (MCP) and proximal interphalangeal (PIP) of the finger. In particular, the device described in ES2558024 requires the adjustment on the metacarpo/phalangeal joint (MCP), the latter having to coincide with the center of the first circular guide. The device described in US20100305717, instead, requires the alignment with the finger joints of both circular guides, acting on linear dovetail guides lockable via screws.

This alignment has several disadvantages, including a long time to wear the device, a procedure dependent by the operator and the possible presence of non-controllable loads on the joints, due to a non-perfect alignment.

The device described in ES2558024 ensures that the force transmitted to the phalanges is perpendicular only on the first phalanx, and only if there are no errors in the alignment of the circular waveguide with the MCP joint of the finger. On the second phalanx the alignment with the PIP joint is automatic, but torques on the phalanx are applied.

In addition, the devices described in ES2558024 and US20100305717 are all mechanisms with a single degree of freedom. This implies having to manually impose the angle of both the MCP and PIP joints, leading to further possible misalignments between anatomical and mechanical joints, and therefore to unwanted parasitic forces, and to constrain the movements of flexion/extension of the phalanges on an imposed trajectory that fixes the relations between the angles of the MCP and PIP joints.

### Summary of the invention

It is therefore a feature of the present invention to provide an exoskeleton device that allows to reduce unwanted stresses on anatomical joints.

It is also a feature of the present invention to provide a said exoskeleton device that allows a dedicated and more precise attuation for each single phalanx of the fingers, in order to adapt during the grip of objects with shape and size not known a priori.

It is a further feature of the present invention to provide a said exoskeleton device that is safe, comfortable and quick to wear, without requiring initial operations of adjustment or calibration.

It is still a feature of the present invention to provide a said exoskeleton device that is adaptable to different anthropometric measurements of the anatomical limbs of a user.

These and other objects are achieved by an exoskeleton device arranged to assist the movement of the phalangeal articulations of a thumb finger, according to claims from 1 to 4.

In this way, the transmission of motion between the exoskeletal structure and phalangeal joint increases its effectiveness and user comfort, since it does not rely on the transmission of tangential forces between the contact surfaces, which may be unreliable and uncomfortable due to slippage between said surfaces in case of high magnitude forces. In addition, the exoskeleton device, once worn on each finger, is a labile structure with 2 degrees of freedom. Being equipped with a single actuator, said exoskeleton device is configured so as an underactuated system with a degree of underactuation. This implies that the movement of the single actuator takes to the variation of both θ₁ and θ₂, at the same time allowing the finger to place itself in any position without generating forces parallel to the axes y and y'. In this way, you have therefore a distribution of the loads on the different phalanges, allowing a better adaptation to the object to be grasped. Moreover, the fact of not requiring a second actuator reduces the weight, the cost and complexity of the exoskeleton.

### Brief description of the drawings

Further characteristic and/or advantages of the present invention are more bright with the following description of an exemplary embodiment thereof, exemplifying but not limitative, with reference to the attached drawings in which:
- Fig. 1 shows, by way of example, a side view of an exoskeleton device, not covered by the invention, in the open configuration;
- Fig. 2 shows, by way of example, a side view of the exoskeleton device of Fig. 1 in the closed configuration;
- Fig. 3 shows, by way of example, a perspective view of the exoskeleton device of Fig. 1 in the open configuration and applied to four fingers of the hand;
- Fig. 4 shows, by way of example, a perspective view of the exoskeleton device of Fig. 3 in the closed configuration;
- Fig. 5 shows a perspective view of an embodiment of the exoskeleton device for the thumb, according to the present invention, together with four exoskeleton devices, not covered by the invention, applied to the other fingers;
- Fig. 6 shows in a first side view the embodiment of the exoskeleton device for the thumb of Fig. 5;
- Fig. 7 shows in a second side view the embodiment of the exoskeleton device for the thumb of Fig. 5.

### Description of a preferred exemplary embodiment

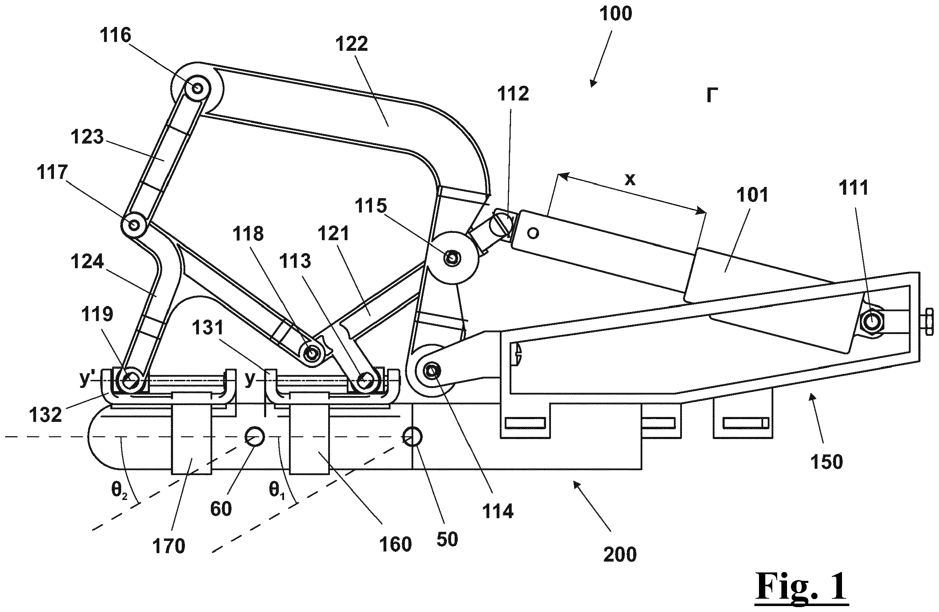

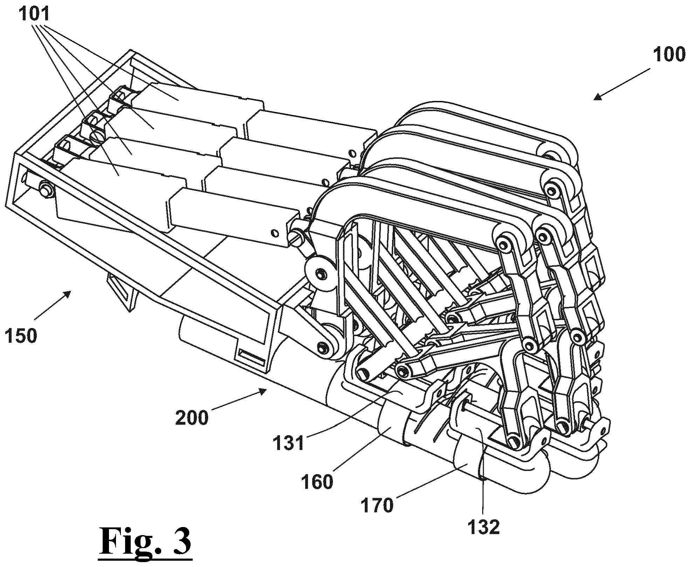

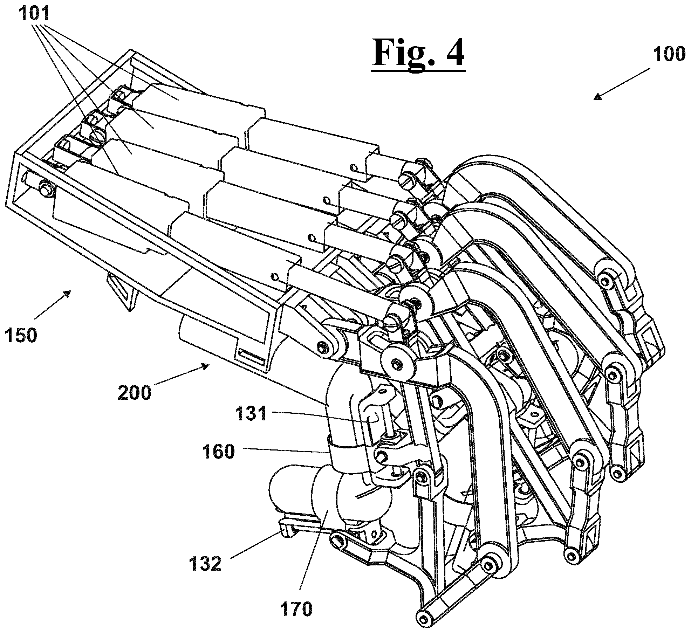

In Figs. 1, 2, 3 and 4, in an example not covered by the present invention, an exoskeleton device 100 is shown adapted to assist the movement of the two phalangeal articulations 50 and 60 of a finger 200 located in a flexion/extension plane Γ.

In particular, the exoskeleton device 100 is adapted to pass between an open configuration and a closed configuration during a rotation θ₁ of the phalangeal articulation 50 about an axis substantially orthogonal to the flexion/extension plane Γ and a rotation θ₂ of the phalangeal articulation 60 about an axis substantially orthogonal to the flexion/extension plane Γ.

Advantageously, the exoskeleton device 100 comprises a metacarpal support 150 integral to a metacarpal portion of a hand, for example the back, and a first support 160 that can be fixed to a first phalanx of the finger 200, for example the proximal phalanx.

Among the metacarpal support 150 and the first support 160 a kinematic chain is provided comprising a linear actuator 101 constrained to the metacarpal support 150 itself from a first rotational joint 111. The actuator 101 is adapted to carry out a stroke x in a radial direction with respect to the rotational joint 111. The chain comprises then a first stiff link 121, connected to the actuator 101 by a second rotational joint 112, and a second stiff link 122, connected to the metacarpal support 150 by a fourth rotational joint 114 and connected to the first stiff link 121 by a fifth rotational joint 115. The chain also comprises a first slide 131 connected to the first support 160 and arranged to carry out a translation with respect to it along an axis y. The slide 131 is also connected to the first stiff link 121 by a third rotational joint 113.

In this example, a combination of the first slide 131 with the third rotational joint 113 causes that they can not make forces parallel to the axis y when the exoskeleton 100 passes in the closed configuration, i.e. tangentially directed to the phalanx on which the device is worn.

In this example, therefore, the actuator 101 can bring in rotation the phalangeal articulation 50, without generating forces tangential with respect to the surface of the phalanx of the finger 200, increasing remarkably the comfort of a user during the use and the efficiency of the transmittion of the forces. In fact, the transmission by friction of forces tangential to the phalanx can result both not much efficient, and not much comfortable, owing to possible relative slipping between the exoskeleton structure and the phalanx itself, in case that the forces are to big. The present invention overcomes this drawback since it does not need such tangential forces for transmitting the motion.

The exoskeleton device 100 is also arranged to assist the movement of the phalangeal articulation 60 of the finger 200 always in the flexion/extension plane Γ.

In particular, the exoskeleton device 100 is adapted to pass between an open configuration and a closed configuration during a rotation θ₂ of the phalangeal articulation 60 about an axis substantially orthogonal to the flexion/extension plane Γ.

Advantageously, the exoskeleton device 100 comprises a first support 160 that can be fixed to a first phalanx of the finger 200, for example to the intermediate phalanx.

Furthermore, the kinematic chain comprises a third stiff link 123, connected to the second stiff link 122 by a sixth rotational joint 116, and a fourth stiff link 124, connected to the third stiff link 123 by a seventh rotational joint 117 and connected to the first stiff link 121 by a eighth rotational joint 118.

The kinematic chain comprises then a second slide 132 connected to the second support 170 and arranged to carry out a translation with respect to it along an axis y'. The second slide 132 is also connected to the fourth stiff link 124 by a ninth rotational joint 119.

Similarly to what happens for the articulation 50, a combination of the second slide 132 with the ninth rotational joint 119 causes that they can not make forces parallel to the axis y' when the exoskeleton 100 passes in the closed configuration.

In this example, once worn on the finger, the exoskeleton device 100 defines a labile kinematic structure with 2 degrees of freedom. This would cause that the variation of the coordinate x by the actuator 101 brings to a variation both of θ₁ and of θ₂, allowing at the same time the finger of place itself in any position not generating forces parallel to axes y and y'.

It is therefore an underactuated system that, in addition to reduce the parassite forces on the anatomicalof joints the user, allows also to use a single actuator for handling both the phalanxes and therefore to not need for an actuator dedicated on the articulation 60. This would cause clear benefits both in economic terms and in terms of weight and encumbrance. Furthermore, the underactuation allows a better distribution of the loads on the different phalanxes, allowing a better matching to the object to grip.

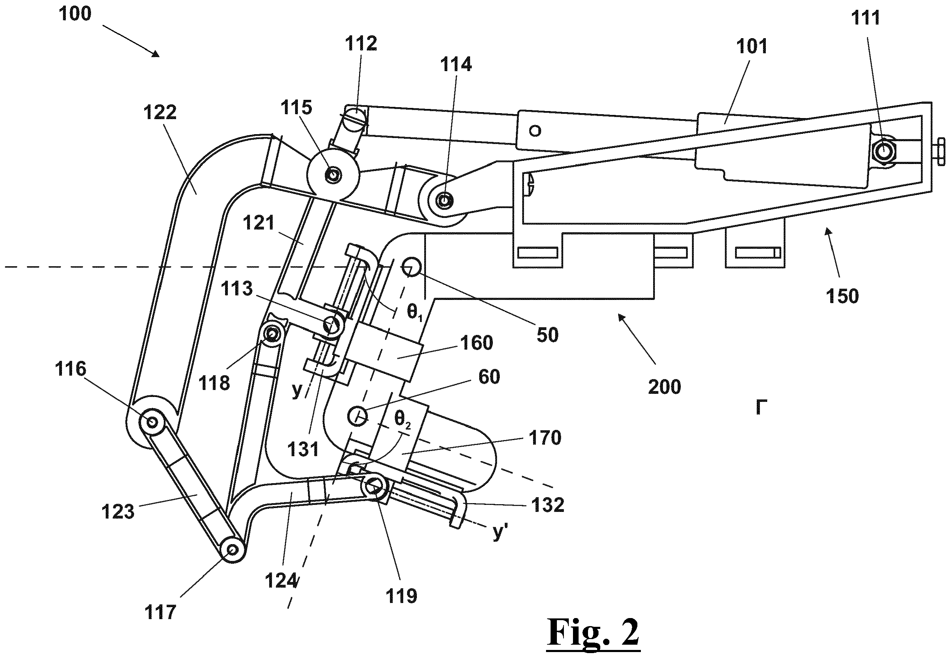

With particular reference to Figs. 1 and 2, in this example, the pivot joints 113 and 119 lay, respectively, on the axes y and y', overlapping to the slides 131 and 132. Such structural aspect causes to avoid jamming of the mechanism during the passage between closed and open configuration, thus making it more fluid the rotation of the joints and more precise the assistance of the exoskeleton 110 to the handling.

Still with reference to Figs. 1 and 2, to avoid mechanical interferences between different components of the kinematic chain during the rotation, the present example provides some structural solutions.

In particular, the L shape of the second stiff link 122 allows it not to overlap, in the closed configuration, to the joint 118 and to the links 121 and 124.

Similarly, the Y shape of the fourth stiff link 124 allows it not to overlap, in the closed configuration, to the proximal interphalangeal articulation and to the slides 131 and 132.

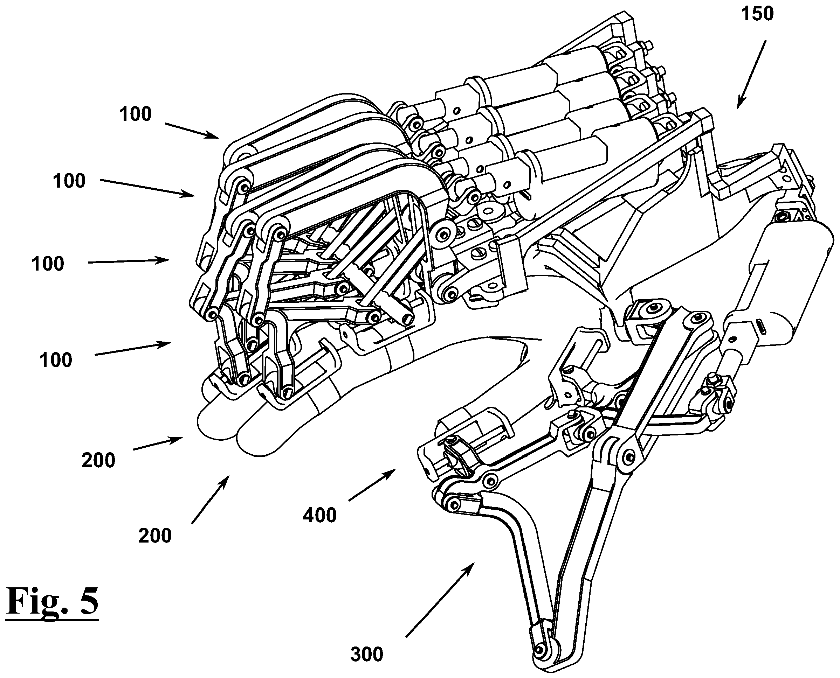

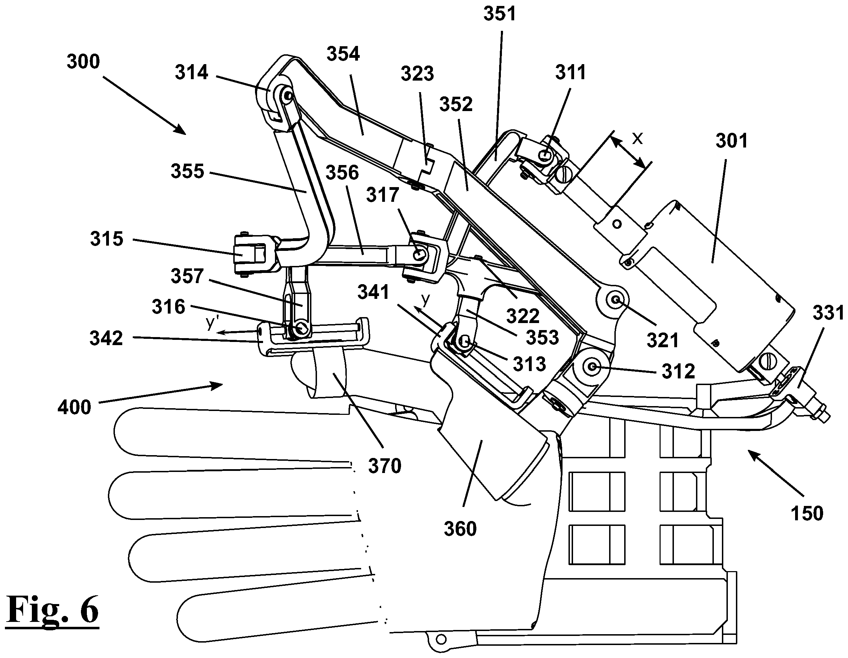

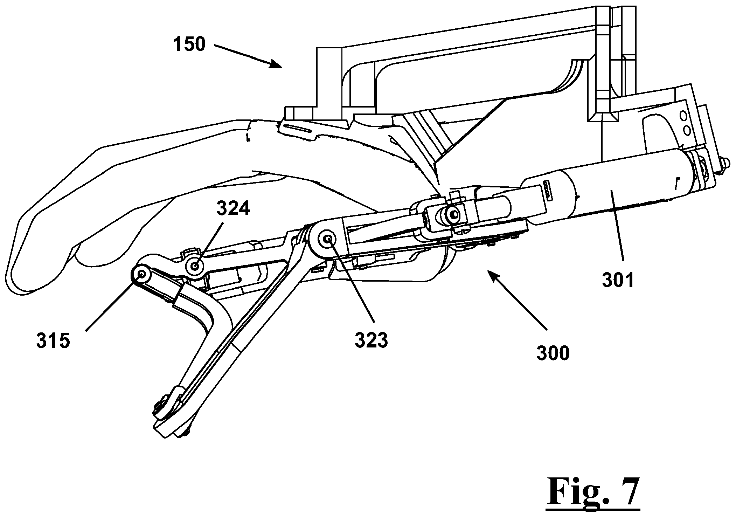

In Figs. 5, 6 and 7, an exoskeleton device 300 applied to the thumb 400 of a hand is shown, according to the present invention.

Kinematically the human thumb 400 differs from the other four fingers of the hand since its movement is not approximable with a plane movement. The thumb is in fact modelable as a serial kinematical chain composed of 5 pivot joints that have skew axes to each other, but suitably directed according to an anatomical model.

The use of universal joints 311, 312, 314, 315 and 317 and of spherical joints 331, 353 and 357 and the introduction of the rotational joint 323 in the kinematic chain of the exoskeleton for thumb 300 has allowed to obtain a three-dimensional workspace, increasing the number of underactuated degrees of freedom of the exoskeleton device 300 with respect to the plane workspace of the exoskeleton 100 used for other fingers and the only degree of underactuation of this exoskeleton 100.

The kinematic chain that forms the exoskeleton for thumb 300 has been designed for being capable, once worn the device, to follow isostatically all the 5 the degrees of freedom (dof) that the thumb has for its kinematic nature. It follows that, all the kinematical system comprising exoskeleton and thumb still has 5 dof. Being operated just one dof of the 5 dof of the system, the latter is underactuated of 4 dof.

Such level of underactuation allows, owing to the particular kinematic chain shown, to guide the thumb in the steps of opening and closing the hand, leaving the finger free to have in each instant the position which more is natural, even if following a conveying direction applied by the device by means of the actuator 301. The device 300 is designed for the application of two forces on the finger thumb 400, one exerted on the metacarpal bone and the other on the distal phalanx. The exoskeleton is in fact constrained to the metacarpal bone by a support 360 suitably shaped and to the distal phalanx by a band 370.

Also in this embodiment 300, as in the exoskeleton 100 for fingers, the forces applied are always perpendicular to the axis of the phalanx on which they are exercitated. This way, all the advantages of comfort and efficiency of transmition of the forces discussed for the embodiment 100 are kept. From the kinematical point of view, in space, this is obtained componing a spherical hinge with a prismatic joint (prismatic joints are represented by the axes y and y' for the respective supports). The spherical hinge allows to have as only constraining reaction a force applied to the centre of the hinge itself, whereas the prismatic joint transmits to the slides 341 and 342, to which is integrally constrained, only the components of this reaction having direction perpendicular to the generic translating direction y.

For simplicity the spherical joint 353 centred in the axis y has been made by the kinematic equivalent of an universal joint 313 centred in the axis y and having a rotation axis in a direction y, and a rotational joint 322 having rotation axis perpendicular to the rotation axes of the universal joint 313 and passing through the centre of the universal joint itself. Similarly, the spherical joint 357 centred in the axis y' has been made by the kinematic equivalent of a universal joint 316 centred in the axis y' and having a rotation axis in a direction y', and a rotational joint 324 having rotation axis perpendicular to the rotation axes of the universal joint 316 and passing through the centre of the universal joint itself.

Finally, the kinematic chain provides that the actuator 301 is mounted between a spherical joint 331 and a universal joint 311, having translational axis always perpendicular to the plane defined by the rotation axes of the universal joint 311. Therefore, the only load which the actuator 301 has to counteract is a force in a direction of the movement, thus preventing possible breaks deriving from the loading of the actuator 301 by means of loads perpendicular to the axis or torques.

## Claims

1. An exoskeleton device (300) arranged to assist the movement of the phalangeal articulations (50,60) of a thumb finger (400), said exoskeleton device (300) arranged to pass between an open configuration and a closed configuration during the rotations θ₁,θ₂ of said phalangeal articulations (50,60), said exoskeleton device (300) comprising:
- a metacarpal support (150) arranged to be integral to a metacarpal portion of a hand;
- a first support (360) having engagement means arranged to engage said first support (360) to a first phalanx of said thumb finger (400);
- a kinematic chain arranged to connect said metacarpal support (150) and said first support (360);
said kinematic chain comprising:
- a linear actuator (301) constrained to said metacarpal support (150) by a first spherical joint (331) and arranged to carry out a stroke x;
- a first stiff link (351) connected to said linear actuator (301) by a first universal joint (311);
- a second stiff link (352) connected to said metacarpal support (150) by a second universal joint (312) and connected to said first stiff link (351) by a first rotational joint (321);
- a second auxiliary stiff link (354) connected to said second stiff link (352) by a second rotational joint (323);
- a first slide (341) connected to said first support (360) and arranged to carry out a translation with respect to it along an axis y, said first slide (341) being also connected to said first stiff link (351) by a second spherical joint (353);
said first slide (341) and said second spherical joint (353) being configured for avoiding the generation of forces parallel to said axis y on said first phalanx of said thumb finger (400), when said exoskeleton device (300) passes between said open configuration and said closed configuration,
said exoskeleton device (300) **characterized in that** it comprises a second support (370) having engagement means arranged to engage said second support (370) to a second phalanx of said thumb finger (400)
**and in that** said kinematic chain comprises furthermore:
- a third stiff link (355) connected to said second auxiliary stiff link (354) by a third universal joint (314);
- a fourth stiff link (356) connected to said third stiff link (355) by a fourth universal joint (315) and connected to said first stiff link (351) by a fifth universal joint (317);
- a second slide (342) connected to said second support (370) and arranged to carry out a translation with respect to it along an axis y', said second slide (342) being connected to said fourth stiff link (356) by a third spherical joint (357);
said second slide (342) and said third spherical joint (357) being configured for avoiding the generation of reactions parallel to said axis y' on said second phalanx of said thumb finger (400), when said exoskeleton device (300) passes between said open configuration and said closed configuration.

2. The exoskeleton device (300), according to claim 1, wherein said second spherical joint (353) comprises a universal joint (313) centred in said axis y and having a rotation axis in a direction y.

3. The exoskeleton device (300), according to claim 1, wherein said third spherical joint (357) comprises a universal joint (316) centred in said axis y' and having a rotation axis in a direction y'.

4. The exoskeleton device (300), according to claim 1, wherein said second stiff link (352) has a L shape configured to avoid that there is an overlap between said second stiff link (352) and other components of said kinematic chain, when said exoskeleton device (300) passes in said closed configuration.

## Patentansprüche

1. Exoskelettvorrichtung (300), die angeordnet ist, um die Bewegung der phalangealen Artikulationen (50, 60) eines Daumenfingers (400) zu unterstützen, wobei die Exoskelettvorrichtung (300) angeordnet ist, um während der Rotationen θ₁,θ₂ der phalangealen Artikulationen (50, 60) zwischen einer offenen Konfiguration und einer geschlossenen Konfiguration zu verlaufen, wobei die Exoskelettvorrichtung (300) Folgendes umfasst:
- eine Mittelhandstütze (150), die angeordnet ist, um einstückig mit einem Mittelhandabschnitt einer Hand zu sein;
- eine erste Stütze (360), die Eingriffsmittel aufweist, die angeordnet sind, um die erste Stütze (360) mit einem ersten Fingerglied des Daumenfingers (400) in Eingriff zu bringen;
- eine kinematische Kette, die angeordnet ist, um die Mittelhandstütze (150) und die erste Stütze (360) zu verbinden;
wobei die kinematische Kette Folgendes umfasst:
- einen linearen Aktor (301), der durch ein erstes Kugelgelenk (331) an der Mittelhandstütze (150) eingeschränkt und angeordnet ist, um einen Hub x durchzuführen;
- ein erstes steifes Verbindungsglied (351), das mit dem linearen Aktor (301) durch ein erstes Universalgelenk (311) verbunden ist;
- ein zweites steifes Verbindungsglied (352), das mit der Mittelhandstütze (150) durch ein zweites Universalgelenk (312) verbunden und mit dem ersten steifen Verbindungsglied (351) durch ein erstes Drehgelenk (321) verbunden ist;
- ein zweites zusätzliches steifes Verbindungsglied (354), das mit dem zweiten steifen Verbindungsglied (352) durch ein zweites Drehgelenk (323) verbunden ist;
- einen ersten Schieber (341), der mit der ersten Stütze (360) verbunden und angeordnet ist, um eine Verschiebung in Bezug darauf entlang einer Achse y durchzuführen, wobei der erste Schieber (341) auch mit dem ersten steifen Verbindungsglied (351) durch ein zweites Kugelgelenk (353) verbunden ist;
wobei der erste Schieber (341) und das zweite Kugelgelenk (353) konfiguriert sind, um die Erzeugung von Kräften parallel zu der Achse y auf dem ersten Fingerglied des Daumenfingers (400) zu vermeiden, wenn die Exoskelettvorrichtung (300) zwischen der offenen Konfiguration und der geschlossenen Konfiguration verläuft,
wobei die Exoskelettvorrichtung (300) **dadurch gekennzeichnet ist, dass** sie eine zweite Stütze (370) umfasst, die Eingriffsmittel aufweist, die angeordnet sind, um die zweite Stütze (370) mit einem zweiten Fingerglied des Daumenfingers (400) in Eingriff zu bringen,
**und dadurch, dass** die kinematische Kette ferner Folgendes umfasst:
- ein drittes steifes Verbindungsglied (355), das mit dem zweiten zusätzlichen steifen Verbindungsglied (354) durch ein drittes Universalgelenk (314) verbunden ist;
- ein viertes steifes Verbindungsglied (356), das mit dem dritten steifen Verbindungsglied (355) durch ein viertes Universalgelenk (315) verbunden und mit dem ersten steifen Verbindungsglied (351) durch ein fünftes Universalgelenk (317) verbunden ist;
- einen zweiten Schieber (342), der mit der zweiten Stütze (370) verbunden und angeordnet ist, um eine Verschiebung in Bezug darauf entlang einer Achse y' durchzuführen, wobei der zweite Schieber (342) mit dem vierten steifen Verbindungsglied (356) durch ein drittes Kugelgelenk (357) verbunden ist;
wobei der zweite Schieber (342) und das dritte Kugelgelenk (357) konfiguriert sind, um die Erzeugung von Reaktionen parallel zu der Achse y' auf dem zweiten Fingerglied des Daumenfingers (400) zu vermeiden, wenn die Exoskelettvorrichtung (300) zwischen der offenen Konfiguration und der geschlossenen Konfiguration verläuft.

2. Exoskelettvorrichtung(300) nach Anspruch 1, wobei das zweite Kugelgelenk (353) ein Universalgelenk (313) umfasst, das in der Achse y zentriert ist und eine Rotationsachse in einer Richtung y aufweist.

3. Exoskelettvorrichtung(300) nach Anspruch 1, wobei das dritte Kugelgelenk (357) ein Universalgelenk (316) umfasst, das in der Achse y' zentriert ist und eine Rotationsachse in einer Richtung y' aufweist.

4. Exoskelettvorrichtung (300) nach Anspruch 1, wobei das zweite steife Verbindungsglied (352) eine L-Form aufweist, die konfiguriert ist, um zu vermeiden, dass es eine Überlappung zwischen dem zweiten steifen Verbindungsglied (352) und anderen Komponenten der kinematischen Kette gibt, wenn die Exoskelettvorrichtung (300) in der geschlossenen Konfiguration verläuft.

## Revendications

1. Dispositif d'exosquelette (300) destiné à faciliter le mouvement des articulations phalangiennes (50, 60) d'un pouce (400), ledit dispositif d'exosquelette (300) étant destiné à passer entre une configuration ouverte et une configuration fermée lors des rotations θ₁, θ₂ desdites articulations phalangiennes (50, 60), ledit dispositif d'exosquelette (300) comprenant :
- un support métacarpien (150) destiné à être intégré à une partie métacarpienne de la main ;
- un premier support (360) comportant des moyens de mise en prise destinés à mettre ledit premier support (360) en prise avec une première phalange dudit pouce (400) ;
- une chaîne cinématique destinée à relier ledit support métacarpien (150) et ledit premier support (360) ;
ladite chaîne cinématique comprenant :
- un actionneur linéaire (301) contraint audit support métacarpien (150) par une première articulation sphérique (331) et destiné à effectuer une course x ;
- une première liaison rigide (351) reliée audit actionneur linéaire (301) par une première articulation universelle (311) ;
- une deuxième liaison rigide (352) reliée audit support métacarpien (150) par une seconde articulation universelle (312) et reliée à ladite première liaison rigide (351) par une première articulation rotoïde (321) ;
- une seconde liaison rigide auxiliaire (354) reliée à ladite deuxième liaison rigide (352) par une seconde articulation rotoïde (323) ;
- un premier coulisseau (341) relié audit premier support (360) et destiné à effectuer une translation par rapport à celui-ci le long d'un axe y, ledit premier coulisseau (341) étant également relié à ladite première liaison rigide (351) par une deuxième articulation sphérique (353) ;
ledit premier coulisseau (341) et ladite deuxième articulation sphérique (353) étant conçues pour éviter la génération de forces parallèles audit axe y sur ladite première phalange dudit pouce (400), lorsque ledit dispositif d'exosquelette (300) passe entre ladite configuration ouverte et ladite configuration fermée,
ledit dispositif d'exosquelette (300) **étant caractérisé en ce qu'**il comprend un second support (370) comportant des moyens de mise en prise destinés à mettre ledit second support (370) en prise avec une seconde phalange dudit pouce (400)
**et en ce que** ladite chaîne cinématique comprend en outre :
- une troisième liaison rigide (355) reliée à ladite seconde liaison rigide auxiliaire (354) par une troisième articulation universelle (314) ;
- une quatrième liaison rigide (356) reliée à ladite troisième liaison rigide (355) par une quatrième articulation universelle (315) et reliée à ladite première liaison rigide (351) par une cinquième articulation universelle (317) ;
- un second coulisseau (342) relié audit second support (370) et destiné à effectuer une translation par rapport à celui-ci le long d'un axe y', ledit second coulisseau (342) étant relié à ladite quatrième liaison rigide (356) par une troisième articulation sphérique (357) ;
ledit second coulisseau (342) et ladite troisième articulation sphérique (357) étant conçues pour éviter la génération de réactions parallèles audit axe y' sur ladite deuxième phalange dudit pouce (400), lorsque ledit dispositif d'exosquelette (300) passe entre ladite configuration ouverte et ladite configuration fermée.

2. Dispositif d'exosquelette (300) selon la revendication 1,
ladite seconde articulation sphérique (353) comprenant une articulation universelle (313) centrée dans ledit axe y et présentant un axe de rotation dans une direction y.

3. Dispositif d'exosquelette (300) selon la revendication 1,
ladite troisième articulation sphérique (357) comprenant une articulation universelle (316) centrée dans ledit axe y' et présentant un axe de rotation dans une direction y'.

4. Dispositif d'exosquelette (300), selon la revendication 1,
ladite deuxième liaison rigide (352) présentant une forme en L conçue pour éviter qu'il existe un chevauchement entre ladite deuxième liaison rigide (352) et d'autres composants de ladite chaîne cinématique, lorsque ledit dispositif d'exosquelette (300) passe dans ladite configuration fermée.
